# EUROPÄISCHE PATENTSCHRIFT

(11) **EP 1 267 755 B1**
(45) Veröffentlichungstag und Bekanntmachung des Hinweises auf die Patenterteilung: **24.05.2006**
(21) Anmeldenummer: 01919410.9
(22) Anmeldetag: 29.03.2001
(51) Int. Cl.: A61F 2/44, A61F 2/30, A61F 2/46

(54) **HÖHENVARIIERBARES WIRBELKÖRPERIMPLANTAT UND BETÄTIGUNGSINSTRUMENTENSET DAFÜR**
VARIABLE-HEIGHT VERTEBRAL IMPLANT AND INSTRUMENT KIT FOR MANIPULATION THEREOF
IMPLANT DE CORPS VERTEBRAL DE HAUTEUR VARIABLE ET KIT D'INSTRUMENTS POUR LE MANIPULER

(30) Priorität: 31.03.2000 DE 20005958 U
(43) Veröffentlichungstag der Anmeldung: 02.01.2003
(73) Patentinhaber: Königsee Implantate und Instrumente zur Ostheosynthese GmbH, 07426 Königsee (DE)
(72) Erfinder: BÖHM, Heinrich, 99425 Weimar (DE); BUSCH, Thomas, 07422 Bad Blankenburg (DE); ORSCHLER, Erich, 96472 Rödental (DE)
(74) Vertreter: Kruspig, Volkmar
(86) Internationale Anmeldenummer: PCT/EP2001/003611
(87) Internationale Veröffentlichungsnummer: WO 2001/072246

(56) Entgegenhaltungen:
- EP-A- 0 832 622
- WO-A-92/01428
- DE-A- 3 023 942
- DE-A- 19 803 700
- US-A- 4 502 160
- US-A- 5 290 312
- US-A- 5 405 391

## Beschreibung

Die Erfindung betrifft eine Implantatvorrichtung gemäß Oberbegriff des Patentanspruchs 1.

Aus der DE 43 28 062 A1 ist ein Stützstab mit seitlich aufschiebbaren Implantatkörpern bekannt. Um ein Aufschieben zu ermöglichen, besitzen die Implantatkörper eine seitliche Nut, wobei eine Oberflächenstruktierung in Form einer Verzahnung der Fixierung der Flächen des Implantatkörpers untereinander sowie darüber hinaus dem Lageerhalt bezüglich des bzw. der Wirbelkörper dient.
Dadurch, daß die dort gezeigten Implantatkörper in unterschiedlicher Höhe vorgefertigt und auf den Stützstab aufschiebbar sind, kann in gewisser Weise eine Höhenanpassung erfolgen. Die Handhabung des Implantats ist jedoch unter operativen Bedingungen außerordentlich erschwert und die Herstellungskosten sind hoch.

Aus der EP 0 302 719 ist ein Implantat in U-Form bekannt, wobei dort Öffnungen vorgesehen sind, die der Aufnahme von Knochenzement dienen. Weitere Öffnungen können als Aufnahme für eine Schraube vorhanden sein, um das Implantat unmittelbar im Wirbel zu fixieren. Als Material wird für das gezeigte Implantat auf kohlefaserverstärkten Kunststoff verwiesen.

Der Implantat-Platzhalter nach EP 0 268 115 A1 besitzt ein Zylindermantelelement mit einer Vielzahl von rautennetzförmigen Ausnehmungen. Ein dort vorgesehener Ring am oberen und unteren Ende des Zylindermantelelements verhindert ein unerwünschtes zu tiefes Eindringen des Implantats in den Wirbel. Dem gleichen Zweck dient eine zusätzlich vorgesehene Bodenplatte. Das Zylindermantelelement ist geschlossen und besitzt neben den Rautenöffnungen keine weiteren zum Einbringen von Knochenzement, so daß auch hier Schwierigkeiten bei der operativen Handhabung bestehen mit Nachteilen beim Knochenzementeinpressen.

Die DE 196 15 938 A1 offenbart eine Wirbelsäulen-Stützvorrichtung zur Durchführung einer intersomatischen Arthrodese. Die Stützvorrichtung besteht aus Titan und ist flach und langgestreckt ausgebildet und in ihrem Mittelbereich verbreitert. Die Kanten besitzen mit Ausnahme des Mittelbereichs ein Sägezahnprofil. Die Gestalt der vorgestellten Stützvorrichtung ist der eines offenen Ringes ähnlich, wobei durch das Aufbiegen der Endbereiche die Stützvorrichtung durchmesserseitig verschiedenen Anwendungsfällen angepaßt werden kann.

Ein höhenvariierbarer Wirbelkörperersatz mit einer Hülse und einem in axialer Richtung verschiebbaren ersten Widerlagerkörper bzw. einem zweiten Widerlager ist aus der DE 44 09 392 A1 bekannt. Zum Bewegen der Widerlagerkörper untereinander ist eine Gewindevorrichtung vorgesehen. Ein ähnliches Prinzip ist in der DE 44 23 257 A1 gezeigt.

Eine Gewindeverstellbarkeit wird auch in der DE 195 19 101 A1 erwähnt, wobei dort an den Enden der Hülsenteile des Wirbelkörperersatzes zackenförmige Ausnehmungen vorgesehen sind, um ein radiales Arretieren gegenüber den Wirbeln zu realisieren. Die zylindrischen Teile des Wirbelkörperersatzes besitzen eine Vielzahl von Ausnehmungen, um das Durchwachsen mit Körpermaterial zu verbessern. Das Einsetzen des Wirbelkörperersatzes aus DE 195 19 101 A1 ist jedoch außerordentlich problematisch, da hierfür ein entsprechendes Aufspreizen der Wirbel erfolgen muß. Darüber hinaus besitzt das bekannte Implantat eine zu große metallische Masse mit entsprechenden nachteiligen postoperativen Folgen.

Die DE 195 09 317 A1 zeigt ein Implantat zum Einsetzen zwischen Wirbelkörper der Wirbelsäule als Platzhalter, wobei zwei endständige Implantatteile vorgesehen sind, die mit einem dazwischen befindlichen mittigen Implantatteil, das mit den endständigen Implantatteilen drehbar verbunden ist, wechselwirkt. Die gezeigten Implantatteile sind als rohrförmige Hülse bzw. als Stirnring ausgebildet. Durch vorgesehene Öffnungen kann Knochenzement eingebracht werden. Diese Öffnungen sind jedoch nur dann optimal zum Einbringen von Knochenzement geeignet, wenn diese zur Deckung gebracht werden, was jedoch bei dem gewählten Verstellprinzip problematisch ist.

Hinsichtlich der Verstellbarkeit sei noch auf die US 5,236,460 verwiesen, die eine Teleskopanordnung eines Implantats zeigt, wobei mit Hilfe eines speziellen Werkzeuges ein aushärtendes Material in den Teleskopraum verbracht werden kann. Die EP 0 637 439 A1 hingegen zeigt ein Implantat mit einem Keilgetriebe zur Höhenverstellung. Zusätzlich können dort Arretierungszacken herausgeschwenkt werden, um das Implantat nach dem Einbringen zwischen den Wirbeln zu verkrallen.

Bei der US 5,290,312 handelt es sich um ein höhenvariierbares Wirbelkörperimplantat mit zwei U-förmigen Körben, wobei der innere Korb vom äußeren Korb umgriffen und geführt wird. Die dort gezeigten Körbe werden jedoch über Schrauben im Wirbel punktuell und nur einseitig fixiert. Weiterhin muß zum Aufspreizen eine Zange oder dergleichen Instrument eingesetzt werden, da das Implantat selbst keine entsprechenden Verstellmittel aufweist, so daß minimal invasive Eingriffe nicht möglich sind.

Aus der gattungsbildenden DE 198 03 700 A1 ist ein höhenvariierbares Wirbelkörperimplantat mit zwei teleskopartig ineinander geführten Körben, welche verstellbar sind, vorbekannt, wobei der dortige innere und äußere Korb in einer vorgegebenen Endlage gegeneinander fixierbar sind. Die dort vorgesehene Schraube zur Höhenverstellung verbleibt grundsätzlich am bzw. im Implantat.

Unter Berücksichtigung des aufgezeigten Standes der Technik besteht die Aufgabenstellung der Erfindung darin, eine weiterentwickelte Implantatvorrichtung mit Betätigungsinstrumentenset anzugeben, das bei geringstem Materialaufwand eine ausgezeichnete Stabilität aufweist und welches nicht nur als üblicher Platzhalter Anwendung findet, sondern das auch zum Spreizen der Wirbel und zum dauerhaften Korrigieren von Fehlstellungen eingesetzt werden kann, so daß die operative Handhabung sehr einfach ist. Weiterhin soll das höhenvariierbare Wirbelkörperimplantat optimierte Öffnungen zum Einbringen von Knochenzement besitzen, wobei auch das Verwachsen zu gewährleisten ist. Auch soll das Implantat für minimal invasive Eingriffe geeignet sein.

Die Lösung der Aufgabe der Erfindung erfolgt mit einem Gegenstand gemäß Definition nach Patentanspruch 1, wobei die Unteransprüche zweckmäßige Ausgestaltungen und Weiterbildungen umfassen.

Erfindungsgemäß ist die spezielle Verstelleinrichtung. Hierfür besitzt der innere Korb vorzugsweise im Bereich des die Schenkel verbindenden Teiles ein sich in Längsrichtung erstreckendes Langloch mit einer Verzahnung. Hier kann im Zusammenwirken mit dem speziellen Instrumententeil, welches eine komplementäre Verzahnung besitzt, eine Relativbewegung und Verstellung der Körbe zueinander erreicht werden. Operationsseitig wird das Implantat mit rückgefahrenem Korb eingesetzt, wobei durch Einführen des Instrumententeils das gewünschte Aufspreizen, aber auch Zurückfahren erfolgen kann. Nach Erhalt der Endposition wird mittels mindestens einer Feststellschraube diese Position fixiert. Hierfür ist ein zweites Langloch neben dem ersten Langloch im inneren Korb ausgebildet.

Gemäß einem weiteren Gedanken besitzen die Körbe Wirbelstützflächen-Stege, die jeweils am Kopf des Innen- oder am Fuß des Außenkorbs angebracht sind. Diese Stege sind so konzipiert, daß sie auch vom Arzt getrennt bzw. Teile davon herausgetrennt werden können.

Fertigungsseitig wird bevorzugt die Fräs-/Erodiertechnik aus einem Rohling angewendet, so daß sich die Stabilität des Implantats erhöht.

Es sei angemerkt, daß unter U-förmig auch eine mehreckige Form, eine halbkreis- oder kreisförmige Zylinderform oder dergleichen zu verstehen ist, d.h. wesentlich soll bei den erfindungsgemäßen Körben die Möglichkeit des teleskopartigen Ineinanderführens und der gegenseitigen Abstützung der Seitenwände sein, wobei gewährleistet ist, daß sich eine durchgehende seitliche Öffnung einstellt.

Beim Erfindungsgegenstand wird, wie oben dargestellt, also von einem ersten, inneren Korb ausgegangen, der von einem zweiten, äußeren U-förmigen Korb umgriffen und geführt ist. Die Schenkel des inneren und äußeren Korbes sind so ausgerichtet, daß sich eine durchgehende seitliche Öffnung ergibt. Diese durchgehende seitliche Öffnung zeigt bei der Anwendung zum Operateur und ermöglicht sowohl das Einführen des Werkzeugs zur dargelegten Höhenverstellung resp. zur Arretierung als auch das optimale Einbringen von Knochenzement.

Um die Stabilität des Wirbelkörperimplantats zu erhöhen, besitzen die Schenkel des inneren und äußeren Korbes an ihren Schenkelenden jeweils einen nach innen gerichteten Krümmungsabschnitt. Hierdurch wird trotz der erwähnten durchgehenden seitlichen Öffnung bei einwirkenden Druck- und/oder Rotationskräften die gewünschte vorgegebene Lagezuordnung erhalten, ohne daß weitere stabilisierende Mittel notwendig werden.

Ergänzend besteht die Möglichkeit, daß mindestens im Bereich der Krümmungsabschnitte der sich gegenüberliegenden Flächen der Körbe eine vorzugsweise geprägte Rastverzahnung vorhanden ist, um ein Verrutschen oder unerwünschtes Verdrehen der Körbe gegeneinander auszuschließen.

Im Bereich des die Schenkel verbindenden Teiles der U- oder C-förmigen Körbe sind Bohrungen zur Aufnahme mindestens einer Feststellschraube angeordnet, wobei die entsprechende Bohrung des äußeren Korbes ein Gewinde umfaßt.

Die Höhenvariierbarkeit des Wirbelkörperimplantats wird wie dargelegt dadurch realisiert, daß der innere Korb vorzugsweise im Bereich des die Schenkel verbindenden Teiles ein sich in Längsrichtung erstreckendes erstes Langloch mit einer Verzahnung aufweist, um hier im Zusammenwirken mit dem Instrument eine Relativbewegung und entsprechende Verstellung zwischen den Körben zu erreichen.
Ein weiteres, zweites Langloch ist vorzugsweise dem ersten Langloch benachbart vorgesehen und dient dem Feststellen oder Zwischenfixieren der Einstellposition mittels Feststellschraube.

Um das Eindringen der Arretierungsvorsprünge bis zum Inanschlagkommen der Wirbelstützflächen bezogen auf die angrenzenden Wirbel zu erleichtern, ohne starke Knochenschädigungen hervorzurufen, besitzen die Arretierungsvorsprünge, die beispielsweise Dreiecksform aufweisen, einen Schneidschliff.

Die Arretierungsvorsprünge und Wirbelstützflächen oder Stege, die jeweils an einer der Stirnseiten des ersten und des zweiten Korbes vorgesehen sind, werden zweckmäßigerweise alternierend angeordnet, um die gewünschte Planlage des Implantats sicherzustellen.

In einer Ausführungsform der Erfindung können die Körbe über ein Verbindungsteil stapelbar ausgeführt werden. Hierbei besteht die Möglichkeit, ausgehend von einem wiederum speziellen Mittelteil beidseitig ausfahrbare Körbe vorzusehen.

Die Grund- und/oder Deckflächen der Körbe können eine von der Parallelen abweichende, den anatomischen Gegebenheiten angepaßte Winkellage respektive Form aufweisen.

Ausgestaltend besteht die Möglichkeit, die Körbe in gekrümmter Form auszuführen, die dann entlang einer Kreissegment-Führungsbahn verstellbar sind.

Im Regelfall genügt eine Arretierung des Implantats durch die Spreizung gegenüber den jeweils benachbarten Wirbelkörpern, jedoch ist ausgestaltend die Möglichkeit vorgesehen, das Implantat zusätzlich, z.B. durch Schrauben zu verankern, so daß alle Kräfte, die bei natürlichen Bewegungsfolgen des menschlichen Körpers auftreten, sicher abgeleitet werden können.

Das Betätigungsinstrumentenset für das vorgestellte höhenvariierbare Wirbelkörperimplantat umfaßt also das Instrumententeil mit langgestrecktem Führungsstab und einem Gewindeende. Das Instrumententeil mit Außenverzahnung ist zur Aufnahme des Führungsstabs hohlzylindrisch ausgebildet und weist am der Verzahnung gegenüberliegenden Ende einen Handgriff mit einer auf den Führungsstab wirkenden Rändelschraube auf.

Zusätzlich ist eine Faßzange vorgesehen, welche Klemmbacken besitzt, die in entsprechende Ausnehmungen eingreifen, die an den Implantat-Körben vorgesehen sind.

An der Faßzange können Navigationshilfen zur Lage- bzw. Bilderkennung angebracht sein. Ebenso können an der Faßzange Mittel zum leichteren Positionieren eines Schraubendrehers mit Schraubenhalterung und/oder dem Führungsstab angeordnet werden.

Mit Hilfe des Implantats und dem Betätigungsinstrumentenset ist ein endoskopisch assistiertes Einsetzen des Implantats in minimal invasiver Operationstechnik möglich.
Der Patient befindet sich in Bauchlage, wobei ein operativer Zugriff über die jeweils aufgespreizten Rippen erfolgt.

Das Implantat wird wie folgt vorbereitet. Zunächst wird mindestens eine Feststellschraube in die mindestens eine im äußeren Korb vorgesehene Gewindebohrung eingebracht. Hier wird diese Bohrung gewählt, die im Bereich eines weiteren, zweiten Langlochs liegt, das im inneren Korb ausgebildet ist. Diese Feststellschraube bleibt jedoch zunächst locker, so daß die gewünschte Verschiebbarkeit der Körbe erhalten ist. Nunmehr wird der Führungsstab in die Gewindebohrung des äußeren Korbes eingesetzt, und zwar in diejenige, welche sich in einer Position unterhalb des ersten Langlochbereichs befindet. Anschließend wird das Instrumententeil mit Außenverzahnung über den Führungsstab geschoben und hiernach durch Handgriffdrehung und Verzahnungseingriff die rotatorische in eine translatorische Bewegung umgesetzt.

Mit zurückgefahrenem Korb und unter Nutzung der Faß- oder Haltezange kann dann das Implantat eingesetzt werden. Eine entsprechende Darstellung auf der Faßzange zeigt die Extensionsrichtung des Korbes und somit auch die craniale Implantationsseite an. Das Implantat kann also im vorstehend beschriebenen Sinn in den Corporectomideffekt eingebracht und dort entsprechend in bezug auf die Körperachse ausgerichtet werden. Über das Aufspreizen mittels Instrumententeil mit Außenverzahnung wird dann das Implantat fixiert und es erfolgt die Sicherung der jeweiligen Höhenposition über die Rändelschraube am Instrument, um dann mit Hilfe eines Schraubendrehers die wenigstens eine Feststellschraube anzuziehen.

Bei einer bevorzugten Ausführungsform sind die verwendeten bzw: einzusetzenden Schrauben farblich unterschiedlich gestaltet, bevorzugt rot, gelb und grün, um über diese sich ergebende Ampelfunktion eine Betätigungsabfolge vorzugeben und Verwechslungen auszuschließen.
Es liegt im Sinne der Erfindung, nicht nur eine, sondern mehrere Feststellschrauben anzuordnen. Auch ist es sinnvoll, am Führungsstab eine Kennzeichnung vorzusehen, die es dem Operateur ermöglicht, die exakte Position des Instruments mit Verzahnung zu erkennen, um ein unerwünschtes Abscheren oder Beschädigen des Langloch-Verzahnungsabschnitts auszuschließen. Hierfür kann der Handgriff des Instrumententeils in Fortsetzung der hohlzylindrischen Ausbildung durchgehend ausgebildet sein und am Führungsstab eine Kerbe oder dergleichen eingebracht werden, die mit der Soll-Lage aus dem Handgriffende heraustritt und damit sichtbar oder fühlbar wird.

Es hat sich gezeigt, daß in überraschender Weise die zackenartigen Arretierungsvorsprünge, aber auch die Wirbelstützflächen das Implantat ausreichend gegen die beim Anziehen der Feststellschrauben wirkenden Kräfte sichert.

Die Erfindung soll nachstehend anhand eines Ausführungsbeispiels sowie unter Zuhilfenahme von Figuren näher erläutert werden.

Hierbei zeigen:
- Fig. 1; 2: eine erste Ausführungsform von Außenkorb und Innenkorb in verschiedenen Ansichten und zungenartigen Wirbelstützflächen;
- Fig. 3; 4: eine zweite Ausführungsform von Außenkorb und Innenkorb in verschiedenen Ansichten mit Stegen gleicher Dimension als Wirbelstützflächen;
- Fig. 5; 6: eine dritte Ausführungsform jedoch mit C-Außenkorb und C-Innenkorb in verschiedenen Ansichten mit Stegen als Wirbelstützflächen;
- Fig. 7: eine Ansicht des Instrumententeils mit Außenverzahnung;
- Fig. 8: eine Darstellung des Führungsstabs;
- Fig. 9: Darstellungen zur Handhabung der Faßzange;
- Fig. 10: Darstellungen zur Handhabung des Betätigungsinstrumentensets; und
- Fig. 11: Darstellungen zur Höhenverstellung mittels Instrumententeil mit Außenverzahnung.

Wie aus den Figuren ersichtlich, umfaßt grundsätzlich der innere erste Korb 1 in seinen Schenkeln vorgesehene Ausnehmungen oder Durchbrüche 2. An der Stirnseite des ersten inneren Korbes 1 sind Wirbelstützflächen 3 oder Stege 31 sowie abwechselnd zu diesen Stützflächen 3 oder Steigen 31 sich in Längsrichtung erstreckende zackenartige Arretierungsvorsprünge 4 vorgesehen.
An ihrer Spitze können die Arretierungsvorsprünge einen Schliff 16 zum leichteren Eindringen in die jeweils gegenüberliegende Fläche des Wirbels aufweisen. An einem nach innen gerichteten Krümmungsabschnitt 5 kann eine Aussparung (nicht gezeigt) vorgesehen sein, die mit einer Zunge (nicht gezeigt) des äußeren Korbes 8 durch Verbiegen verklink- oder verkrallbar ist.

Wie aus den Figuren ersichtlich ist, wurde die Fläche und die Form des Implantats so optimiert, daß einerseits eine geringe Materialmenge gegeben ist, andererseits aber auch die mechanische Stabilität den insbesondere postoperativen Anforderungen genügt. Stabilitätserhöhend wirken beispielsweise die erkennbare Anschlagkante 9, aber auch die definierten Radien, die durch bevorzugte Bearbeitung mittels Fräsen oder Erodieren erhalten werden. Im Bereich des die Schenkel 10 verbindenden Teiles 11 ist bei den verschiedenen Ausführungsformen ein sich in Längsrichtung erstreckendes Langloch 12 mit einer Verzahnung 13 ausgebildet. Ein zweites Langloch 14 ist dem ersten Langloch 12 im wesentlichen parallel verlaufend benachbart angeordnet und dient dem Feststellen der gewählten Einstell- oder Verstellposition mittels einer Feststellschraube, die sich in einer Gewindebohrung des zweiten äußeren Korbes 8 befindet.

Im Bereich der Krümmungsabschnitte an oder auf den sich gegenüberliegenden Oberflächen des ersten inneren Korbes 1 und des äußeren zweiten Korbes 8 kann eine Verzahnung vorgesehen sein, die ein unerwünschtes Verschieben oder Verdrehen der Körbe gegeneinander wirksam verhindert.

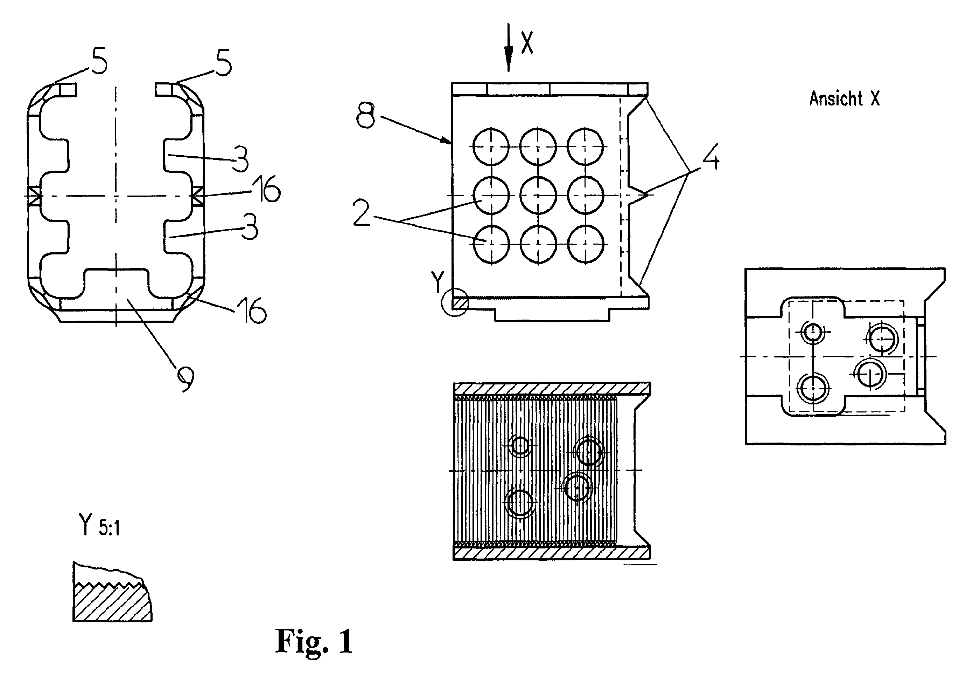

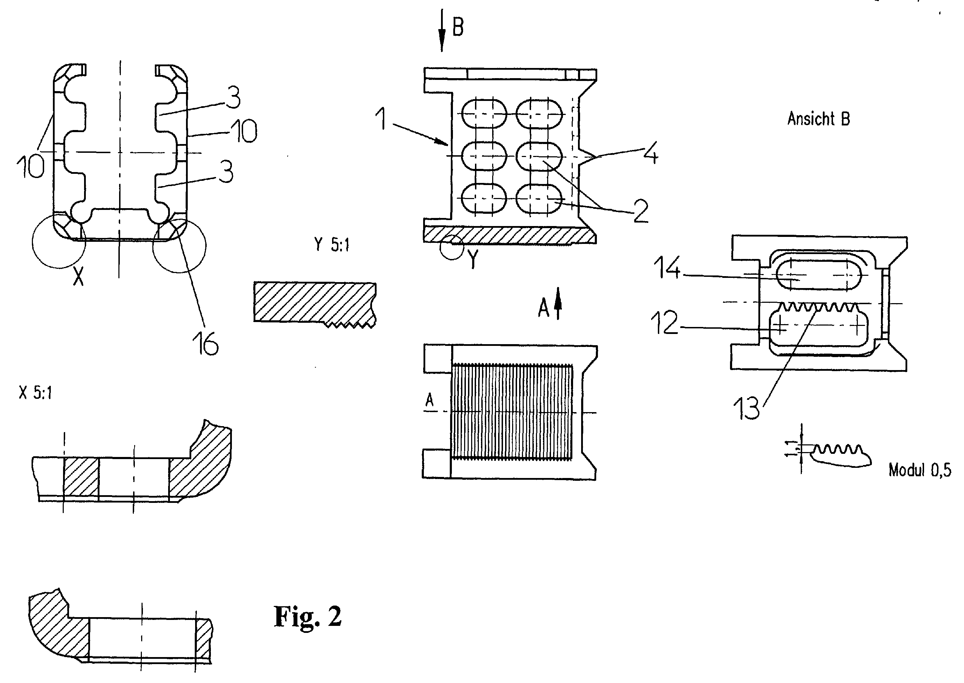

Bei den Fig. 1 und 2 sind die Wirbelstützflächen 3 zungenartig im wesentlich gegenüberliegend ausgebildet, wobei die Zwischenräume die erwähnten Arretierungsvorsprünge 4 aufweisen.

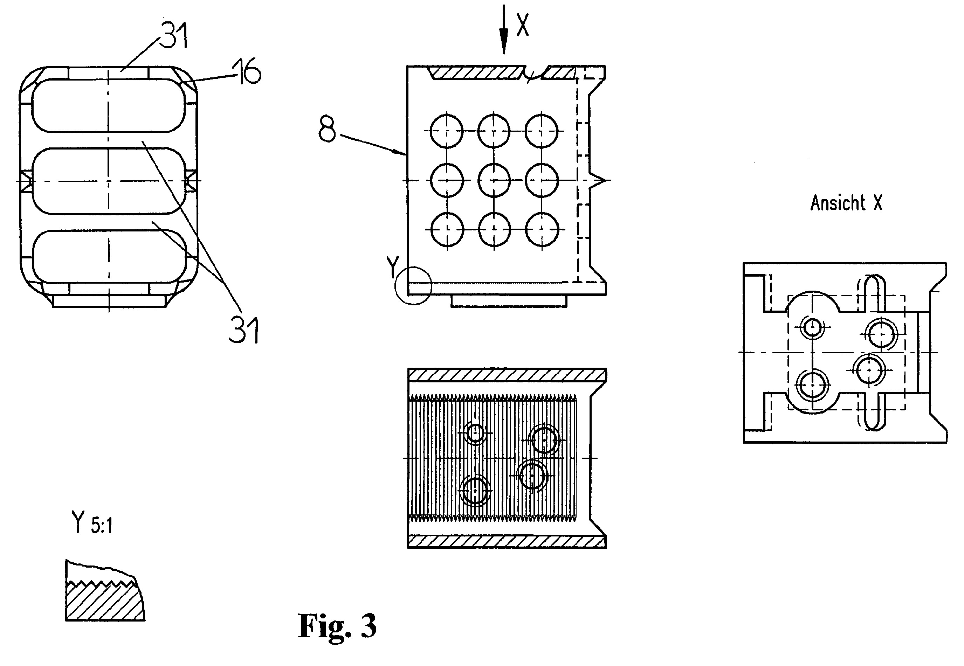

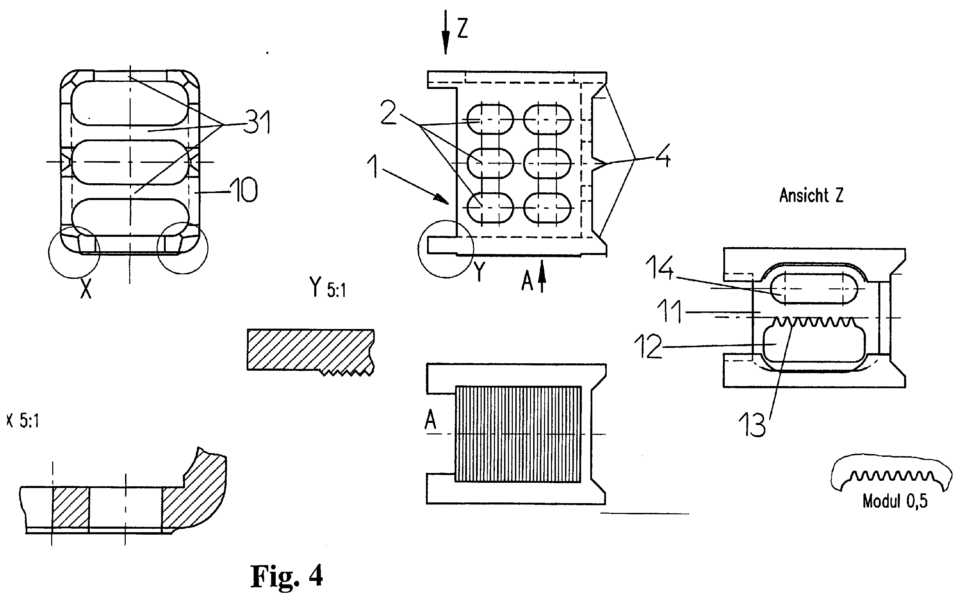

Beim zweiten Ausführungsbeispiel nach den Fig. 3 und 4 sind anstelle der Zungen verbindende Stege 31 relativ geringer Breite vorhanden, wobei z.B. von insgesamt drei Stegen ausgegangen wird und einer der Stege die beiden Schenkel 10 öffnungsseitig verbindet.

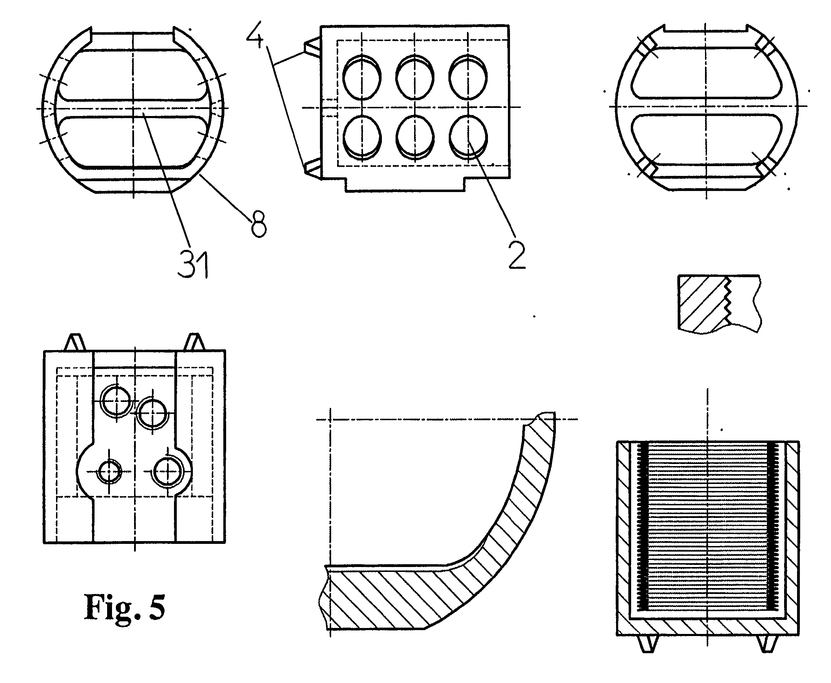

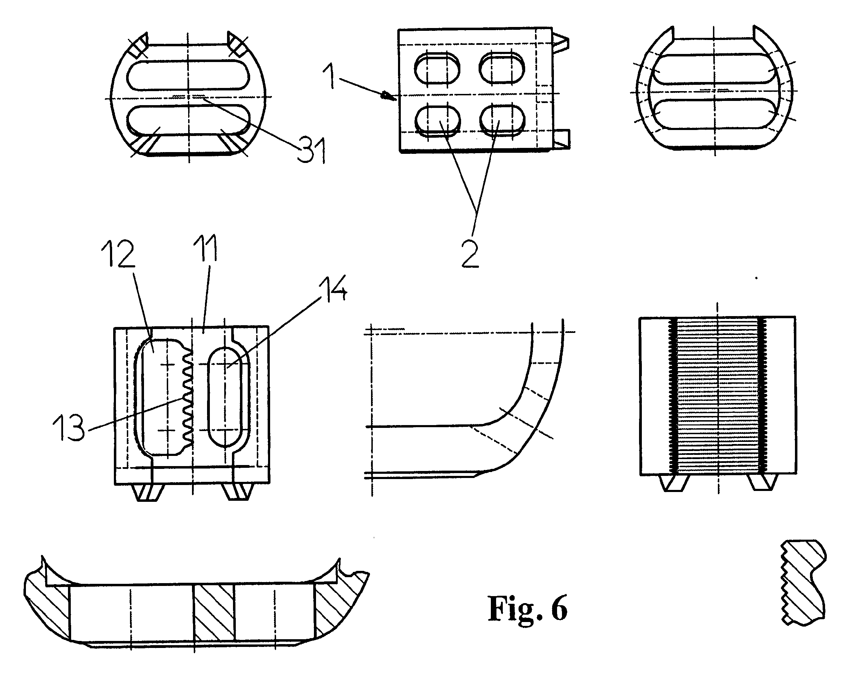

Bei dem Ausführungsbeispiel nach den Fig. 5 und 6 wird von einem C-förmigen Außen- und Innenkorb ausgegangen.

Die Stege 31 erhöhen einerseits die Stabilität der Körbe gegen unerwünschtes Aufspreizen und sorgen andererseits dafür, daß die Flächenpressung zum Wirbelkörper hin reduziert bzw, minimiert wird.

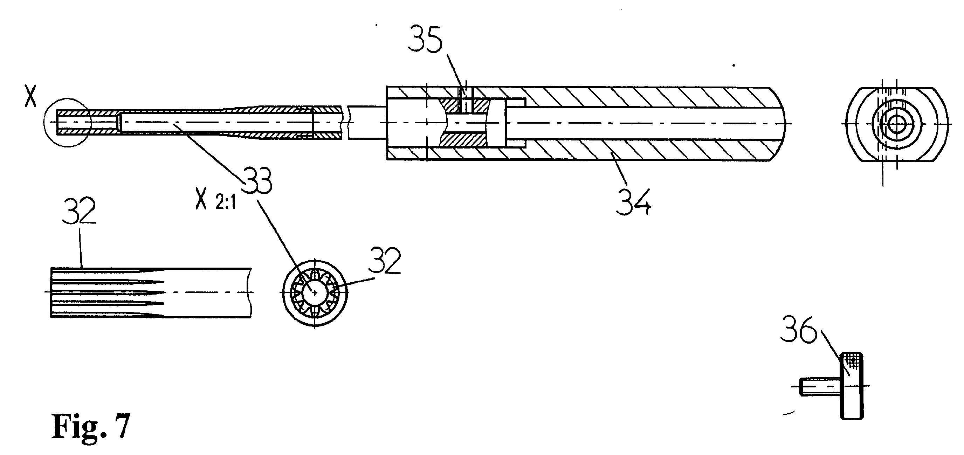

Das in Fig. 7 gezeigte Instrumententeil besitzt eine Außenverzahnung 32, die komplementär zur Verzahnung 13 im ersten Langloch 12 ausgebildet ist. Weiterhin ist das Instrumententeil nach Art eines Hohlzylinders 33 ausgeführt und weist an seinem der Außenverzahnung 32 gegenüberliegenden Ende einen Handgriff 34 auf.

Am Handgriff wiederum ist eine Bohrung 35 mit Gewinde vorgesehen, das zur Aufnahme einer Rändelschraube 36 dient.

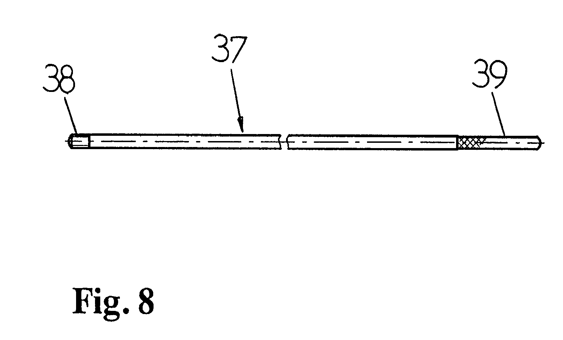

Ein vorgesehener Führungsstab 37 gemäß Fig. 8 besitzt ein Gewindeende 38 sowie einen gerändelten Handhabungsabschnitt 39. Der Hohlzylinder 33 des Instrumententeils mit Außenverzahnung 32 und der Außendurchmesser des Führungsstabs 37 sind so aufeinander abgestimmt, daß das Instrumententeil über den Führungsstab quasi zentriert zur Verzahnung 13 im ersten Langloch 12 bewegt werden kann.

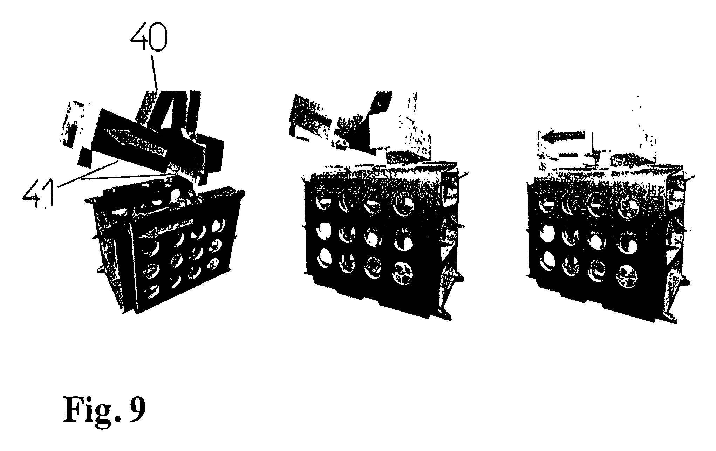

Fig. 9 zeigt in verschiedenen Ansichten den unteren Bereich einer Faßzange 40 mit Klemmbacken 41. Der Pfeil zeigt die Extensionsrichtung des Korbes und somit auch die craniale Implantationsseite an.

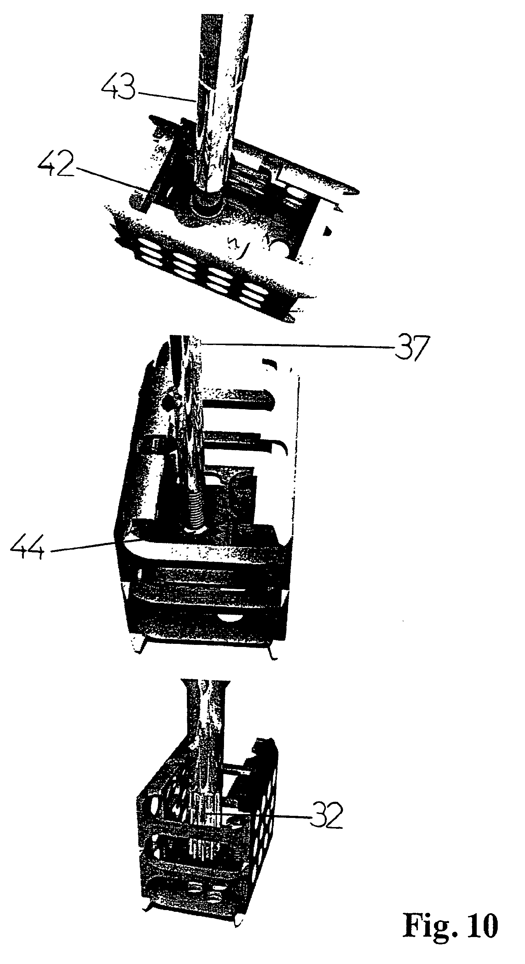

Der obere Bildteil gemäß Fig. 10. läßt das zweite Langloch 14 mit dort eingesetzter Feststellschraube 42 und den unteren Abschnitt eines Schraubendrehers 43 erkennen. Die Feststellschraube 42 wird locker eingedreht.
Gemäß dem mittleren Bild nach Fig. 10 wird in die Gewindebohrung 44, die sich im äußeren Korb unterhalb des Langlochbereichs 12 befindet, der Führungsstab 37 eingedreht. Anschließend wird, wie im unteren Bildteil erkennbar, das Instrumententeil mit Außenverzahnung über den Führungsstab geschoben und in eine Position verbracht, daß die Außenverzahnung in die Verzahnung 13 des ersten Langlochs 12 eingreift.

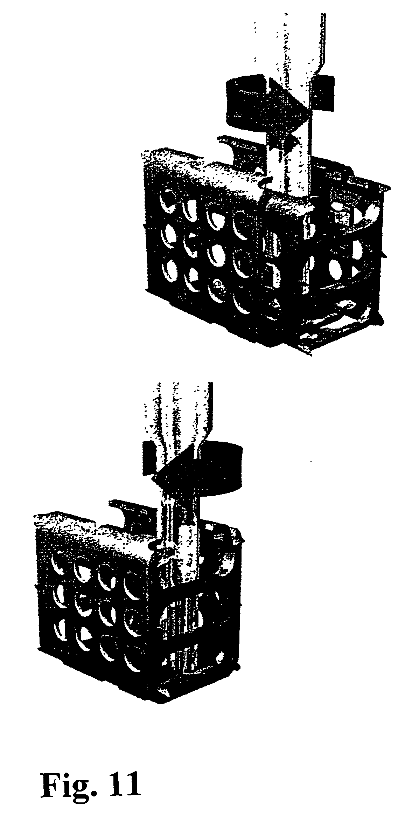

Wie in der Darstellung nach Fig. 11 ersichtlich, kann nun durch Drehen des Instrumententeils mit Außenverzahnung der innere Korb translatorisch bezogen zum äußeren Korb verschoben werden.

Alles in allem gelingt es mit der vorgeschlagenen Implantatvorrichtung, die Wirbelsäule sicher zu stützen, ohne daß die Menge des zur Stützung erforderlichen Fremdkörpermaterials eine kritische Schwelle übersteigt. Die Handhabung des Implantats selbst ist unkompliziert und durch die Höhenvariierbarkeit bezogen auf den Stand der Technik wesentlich erleichtert. Durch die Ausbildung der ineinander geführten Körbe des Implantats aus einem metallischen Material, bearbeitet durch Fräsen und/oder Erodieren, können die Fertigungskosten in Grenzen gehalten werden.

### Bezugszeichenaufstellung

- 1: innerer Korb
- 2: Ausnehmungen, Durchbrüche
- 3: Wirbelstützfläche
- 4: Arretierungsvorsprünge
- 5: Krümmungsabschnitt
- 8: äußerer Korb
- 9: Anschlagkante
- 10: Schenkel
- 11: Verbindungsteil der Schenkel
- 12: erstes Langloch
- 13: Verzahnung
- 14: zweites Langloch
- 16: Schneidschliff
- 31: Stege
- 32: Außenverzahnung
- 33: Hohlzylinder
- 34: Handgriff
- 35: Bohrung im Handgriff
- 36: Rändelschraube
- 37: Führungsstab
- 38: Gewindeende
- 39: gerändelter Handhabungsabschnitt
- 40: Faßzange
- 41: Klemmbacken
- 42: Feststellschraube
- 43: Schraubendreher
- 44: Gewindebohrung

## Patentansprüche

1. Implantatvorrichtung bestehend aus einem Betätigungsinstrumentenset, enthaltend wenigstens ein Instrumententeil, und einem höhenvariierbaren Wirbelkörperimplantat mit einem ersten, im wesentlichen U- oder C-förmigen Korb sowie am Korb ausgebildeten Wirbelstützflächen, wobei der erste Korb ein innerer Korb (1) ist, welcher von einem zweiten, äußeren U- oder C-förmigen Korb (8) umgriffen und teleskopartig geführt wird, wobei weiterhin die Schenkel (10) des inneren und des äußeren Korbes (1; 8) so ausgerichtet sind, daß sich eine durchgehende seitliche Öffnung ergibt, und der innere und äußere Korb (1; 8) in einer vorgegebenen Endlage gegeneinander fixierbar sind, weiterhin jeweils eine der Stirnseiten der Körbe (1; 8) Wirbelstützflächen (3) und/oder Stege (31) sowie sich in Längsrichtung erstreckende, zackenartige Arretierungsvorsprünge (4) besitzen und die Schenkel (10) des inneren und äußeren Korbes (1; 8) an ihren Schenkelenden jeweils einen nach innen gerichteten, stabilitätserhöhenden Krümmungsabschnitt (5) aufweisen, wobei der innere Korb (1) im Bereich (11) des die Schenkel (10) verbindenden Teiles ein sich in Längsrichtung erstreckendes Langloch (12) mit einer einseitigen Verzahnung (13) aufweist,
**dadurch gekennzeichnet, daß**
im Zusammenwirken mit einem eine komplementäre Verzahnung aufweisenden Instrumententeil eine Relativbewegung und Verstellung zwischen den Körben (1; 8) erfolgt, wobei ein Führungsstab (7) mit einem Gewindeende (38) vorgesehen ist, weiterhin das Instrumententeil mit Außenverzahnung (32) zur Aufnahme des Führungsstabs (7) hohlzylindrisch ausgebildet ist und am der Verzahnung (32) gegenüberliegenden Ende dieses einen Handgriff (34) mit auf den Führungsstab (7) wirkender Rändelschraube (36) aufweist.

2. Vorrichtung nach Anspruch 1,
**dadurch gekennzeichnet, daß**
mindestens im Bereich der Krümmungsabschnitte (5) die sich gegenüberliegenden Flächen des inneren und äußeren Korbes (1; 8) eine Rastverzahnung aufweisen und im äußeren Korb in einer Position unterhalb des Langlochbereichs eine Gewindebohrung eingebracht ist.

3. Vorrichtung nach einem der vorangegangenen Ansprüche,
**dadurch gekennzeichnet, daß**
die zackenartigen Arretierungsvorsprünge (4) zum leichteren Eindringen in die Wirbelkörperflächen einen Schneidschliff (16) aufweisen.

4. Vorrichtung nach einem der vorangegangenen Ansprüche,
**dadurch gekennzeichnet, daß**
die jeweils an einer der Stirnseiten des ersten und zweiten Korbes (1; 8) vorgesehenen Wirbelstützflächen (3) und/oder Stege (31) und Arretierungsvorsprünge (4) alternierend angeordnet sind.

5. Vorrichtung nach einem der vorangegangenen Ansprüche,
**dadurch gekennzeichnet, daß**
die Körbe aus einem metallischen Fräs-Erodierteil vorzugsweise aus Titanmaterial bestehen.

6. Vorrichtung nach einem der vorangegangenen Ansprüche,
**dadurch gekennzeichnet, daß**
der erste und der zweite Korb (1; 8) einen mehreckigen, bevorzugt rechteckigen, kreisförmigen oder halbkreisförmigen Querschnitt aufweisen.

7. Vorrichtung nach einem der vorangegangenen Ansprüche,
**dadurch gekennzeichnet,**
**daß** mindestens der äußere, erste Korb Ausnehmungen oder Durchbrüche (2) aufweist.

8. Vorrichtung nach einem der vorangegangenen Ansprüche,
**dadurch gekennzeichnet, daß**
mehrere parallel verlaufende, Stützflächen bildende Stege (31) auch unterschiedlicher Breite vorgesehen sind.

9. Vorrichtung nach einem der vorangegangenen Ansprüche,
**dadurch gekennzeichnet, daß**
die Stege mindestens teilweise heraustrennbar ausgeführt sind und hierfür Sollbruchstellen aufweisen.

10. Vorrichtung nach einem der vorangegangenen Ansprüche,
**dadurch gekennzeichnet, daß**
die Körbe über ein Verbindungsteil stapelbar ausgeführt sind.

11. Vorrichtung nach einem der vorangegangenen Ansprüche,
**dadurch gekennzeichnet, daß**
die Grund- und/oder Deckflächen der Körbe eine von der Parallelen abweichende, den anatomischen Gegebenheiten angepaßte Winkellage aufweisen.

12. Vorrichtung nach einem der vorangegangenen Ansprüche,
**dadurch gekennzeichnet, daß**
die Körbe eine gekrümmte Form aufweisen und entlang einer Kreissegment-Führungsbahn verstellbar sind.

13. Vorrichtung nach einem der vorangegangenen Ansprüche,
**gekennzeichnet durch**
eine zusätzliche Verankerung an oder in den jeweiligen Wirbelkörperflächen.

14. Vorrichtung nach einem der Ansprüche 1 bis 13,
**dadurch gekennzeichnet, daß**
das Betätigungsinstrumentenset eine Faßzange zum Greifen und Orientieren bzw. Ausrichten des Implantats umfaßt.

15. Vorrichtung nach Anspruch 14,
**dadurch gekennzeichnet, daß**
an der Faßzange Navigationshilfen und/oder Mittel zum leichteren Positionieren eines Schraubendrehers mit Schraubenhalterung und/oder des Führungsstabs angeordnet sind.

16. Vorrichtung nach Anspruch 14 oder 15,
**dadurch gekennzeichnet, daß**
zur Höhenverstellung zunächst der Führungsstab in die Gewindebohrung des äußeren Korbes eingesetzt, anschließend das Instrumententeil mit Außenverzahnung über den Führungsstab geschoben und hiernach durch Handgriffdrehung und Verzahnungseingriff die rotarorische in eine translatorische Bewegung umgesetzt wird.

17. Vorrichtung nach Anspruch 16,
**dadurch gekennzeichnet, daß**
mittels der Rändelschraube am Instrumenenteil eine gegebene Höhenposition des Implantats vorläufig fixiert wird, um im Anschluß mit mindestens einer Feststellschraube die Höhenposition endgültig zu sichern, wobei hierfür im äußeren Korb eine weitere Gewindebohrung und im inneren Korb ein weiteres, zweites Langloch, dem ersten Langloch benachbart, vorgesehen ist.

## Claims

1. Implant device consisting of an operational instrument set, which contains at least one instrument part, and a variable-height vertebral-body implant with a first, substantially U- or C-shaped cage as well as vertebra-supporting surfaces formed on the cage, such that the first cage is an inner cage (1) that is encompassed by and telescopically guided within a second, outer U- or C-shaped cage (8), and further such that the limbs (10) of the inner and the outer cage (1; 8) are oriented so that a continuous lateral opening is produced, and the inner and outer cage (1; 8) can be fixed in a prespecified final position with respect to one another, and additionally one end face of each of the cages (1; 8) comprises vertebra-supporting surfaces (3) and/or bridges (31) as well as tooth-like locking projections (4) that extend in the long direction, and each of the limbs (10) of the inner and the outer cage (1; 8) has at its ends an inwardly directed, stability-increasing curved section (5), and such that the inner cage (1) in the region (11) of the part that connects the limbs (10) comprises a slot (12) that extends in the long direction and has a serration (13) along one edge,
**characterized in that**
relative movement and a change in relative position of the cages (1; 8) are produced by interaction with an instrument part having a complementary denticulation, wherein a guide rod (7) with a threaded end (38) is provided and the instrument part with external denticulation (32) is constructed as a hollow cylinder so as to accommodate the guide rod (7), and at the end of said instrument part opposite the denticulation (32) there is a handle (34) with a knurled screew (36) that acts on the guide rod (7).

2. Device according to Claim 1,
**characterized in that** at least in the region of the curved sections (5) the opposed surfaces of the inner and the outer cage (1; 8) comprise a catch-tooth region and in the outer cage a threaded bore is formed in a position below the region of the slot.

3. Device according to one of the preceding claims,
**characterized in that** the tooth-like locking projections (4) comprise a sharpened section (16) to facilitate penetration into the vertebral-body surface.

4. Device according to one of the preceding claims,
**characterized in that** the vertebra-supporting surfaces (3) and/or bridges (31) provided at one of the end faces of each of the first and second cages (1; 8) are arranged in alternation with the locking projections (4) .

5. Device according to one of the preceding claims,
**characterized in that** the cages are made of a metallic part shaped by milling/eroding, preferably of titanium material.

6. Device according to one of the preceding claims,
**characterized in that** the first and second cages (1; 8) havw a polygonal, preferably rectangular, circular or semicircular cross section.

7. Device according to one of the preceding claims,
**characterized in that** at least the outer, first cage comprises apertures or openings (2).

8. Device according to one of the preceding claims,
**characterized in that** several bridges (31) are provided, which are oriented in parallel, form supporting surfaces and can also differ in width.

9. Device according to one of the preceding claims,
**characterized in that** the bridges are designed so that they can be at least partially separated and removed, for which purpose preformed breaking points are provided.

10. Device according to one of the preceding claims,
**characterized in that** the cages are designed so that they can be stacked by way of a connecting part.

11. Device according to one of the preceding claims,
**characterized in that** the floor and/or cover surfaces of the cages are set at an angle that diverges from the parallel so as to be adapted to the anatomical situation.

12. Device according to one of the preceding claims,
**characterized in that** the cages have a curved shape and can be adjusted by movement along a guide path shaped as a segment of a circle.

13. Device according to one of the preceding claims,
**characterized by** additional anchoring at or in the associated vertebral-body surfaces.

14. Device according to one of the claims 1 to 13,
**characterized in that** the operational instrument set comprises gripping forceps for grasping and orientating the implant.

15. Device according to Claim 14,
**characterized in that** on the gripping forceps there are disposed navigation aids and/or means to facilitate positioning of a screwdriver with screw holder, and/or positioning of the guide rod.

16. Device according to Claim 14 or 15,
**characterized in that** for height adjustment the first step is to insert the guide rod into the threaded bore of the outer cage, after which the instrument part with external denticulation is pushed over the guide rod and its handle is then rotated to engage the serration, so that the rotational movement is converted to translational movement.

17. Device according to Claim 16,
**characterized in that** by means of the knurled screw on the instrument part a given height position of the implant is temporarily fixed, and subsequently the height position is secured with at least one fixing screw, for which purpose there are provided in the outer cage an additional threaded bore, and in the inner cage an additional, second slot adjacent to the first slot.

## Revendications

1. Dispositif d'implant composé d'un jeu d'instruments pour le manipuler comprenant au moins une pièce d'instrument et un implant de corps vertébral réglable en hauteur avec un premier panier essentiellement en forme de U ou de C et des surfaces d'appui vertébral formées sur le panier, où le premier panier est un panier intérieur (1), entouré d'un deuxième panier extérieur en forme de U ou de C (8) et télescopiquement guidé, les côtés (10) des paniers intérieur et extérieur (1 ; 8) étant en outre orientés de manière à former une ouverture latérale traversante, et où les paniers intérieur et extérieur (1 ; 8) sont fixables l'un contre l'autre dans une position finale définie, une des faces frontales des paniers (1 ; 8) possédant en outre des surfaces d'appui vertébral (3) et/ou des traverses (31) ainsi que des saillies de blocage (4) en crêtes à extension longitudinale, et où les côtés (10) des paniers intérieur et extérieur (1 ; 8) présentent à chacune de leurs extrémités un segment courbe (5) augmentant la stabilité et dirigé vers l'intérieur, le panier intérieur (1) comportant dans la zone (11) de la pièce de jonction des côtés (10) un trou oblong (12) à extension longitudinale avec une denture unilatérale (13),
**caractérisé en ce que**, en coopération avec une pièce d'instrument présentant une denture complémentaire, un déplacement relatif et un ajustement se produisent entre les paniers (1 ; 8), une tige de guidage (7) étant prévue avec une extrémité filetée (38), et **en ce que** la pièce d'instrument à denture extérieure (32) est de forme cylindrique creuse pour le logement de la tige de guidage (7), et **en ce qu'**à l'extrémité opposée à la denture (32), celle-ci comporte une poignée (34) avec une vis moletée (36) agissant sur la tige de guidage (7).

2. Dispositif selon la revendication 1,
**caractérisé en ce que**, au moins dans la zone des segments courbes (5), les surfaces opposées des paniers intérieur et extérieur (1 ; 8) présentent une denture d'enclenchement, et **en ce qu'**un alésage fileté est présent dans le panier extérieur, dans une position située sous la zone du trou oblong.

3. Dispositif selon l'une des revendications précédentes, **caractérisé en ce que** les saillies de blocage (4) en crêtes présentent une entaille rectifiée (16) pour faciliter leur pénétration dans les surfaces de corps vertébral.

4. Dispositif selon l'une des revendications précédentes, **caractérisé en ce que** les surfaces d'appui vertébral (3) et/ou les traverses (31) et les saillies de blocage (4) respectivement prévues sur une des faces frontales des premier et deuxième paniers (1 ; 8) sont disposées en alternance.

5. Dispositif selon l'une des revendications précédentes, **caractérisé en ce que** les paniers se composent d'une pièce métallique érodée-fraisée, préférentiellement en titane.

6. Dispositif selon l'une des revendications précédentes, **caractérisé en ce que** les premier et deuxième paniers (1 ; 8) présentent une section transversale polygonale, préférentiellement rectangulaire, ou circulaire ou demi-circulaire.

7. Dispositif selon l'une des revendications précédentes, **caractérisé en ce que** le premier panier extérieur au moins présente des évidements ou des percements (2).

8. Dispositif selon l'une des revendications précédentes, **caractérisé en ce que** plusieurs traverses (31) à extension parallèle formant des surfaces d'appui sont également prévues avec des largeurs différentes.

9. Dispositif selon l'une des revendications précédentes, **caractérisé en ce que** les traverses sont détachables au moins partiellement et présentent des points de rupture prédéterminés à cet effet.

10. Dispositif selon l'une des revendications précédentes, **caractérisé en ce que** les paniers sont empilables au moyen d'une pièce de jonction.

11. Dispositif selon l'une des revendications précédentes, **caractérisé en ce que** les surfaces de fond et/ou de sommet des paniers présentent une position angulaire s'écartant de la parallèle, ajustée aux particularités anatomiques.

12. Dispositif selon l'une des revendications précédentes, **caractérisé en ce que** les paniers présentent une forme courbe et son déplaçables le long d'une glissière en arc de cercle.

13. Dispositif selon l'une des revendications précédentes, **caractérisé par** un ancrage complémentaire sur ou dans les surfaces de corps vertébral respectives.

14. Dispositif selon l'une des revendications 1 à 13, **caractérisé en ce que** le jeu d'instruments de manipulation comprend une pince à séquestre pour la saisie et l'orientation ou l'alignement de l'implant.

15. Dispositif selon la revendication 14, **caractérisé en ce que** des aides à la navigation et/ou des moyens pour faciliter le positionnement d'un tournevis à maintien de vis et/ou de la tige de guidage sont disposés sur la pince à séquestre.

16. Dispositif selon la revendication 14 ou 15, **caractérisé en ce que** pour le réglage de hauteur, la tige de guidage est d'abord introduite dans l'alésage fileté du panier extérieur, la pièce d'instrument à denture extérieure est ensuite poussée sur la tige d'instrument, et **en ce que** le mouvement rotatif est enfin converti en mouvement translatif par rotation de poignée et engagement de denture.

17. Dispositif selon la revendication 16, **caractérisé en ce qu'**une position de hauteur donnée de l'implant est provisoirement fixée au moyen de la vis moletée sur la pièce d'instrument, pour ensuite fixer définitivement la position en hauteur avec une vis de blocage au moins, un autre alésage étant prévu dans le panier extérieur et un autre trou oblong, voisin du premier trou oblong, dans le panier intérieur, à cet effet.
